# EUROPEAN PATENT APPLICATION

(11) **EP 4 678 768 A1**
(43) Date of publication of application: **14.01.2026**
(21) Application number: 24382735.9
(22) Date of filing: 09.07.2024
(51) Int. Cl.: C12Q 1/6886

(54) **SIGNATURE FOR PROGNOSIS OF METASTASIS RISK AND CHEMOTHERAPY RESPONSE**

(71) Applicant: Fundación de la Comunidad Valenciana Centro de Investigación Principe Felipe, 46012 Valencia (ES)
(72) Inventor: RODRÍGUEZ VITA, Juan, 46012 Valencia (ES); DE ANGELIS RIGOTTI, Francesca, 46012 Valencia (ES)
(74) Representative: Isern Patentes y Marcas S.L.

(57) **Abstract**

The present invention relates to method for the identification of subjects with increased risk of suffering of metastasis and/or being bad responder to chemotherapy, by the determination of the expression level of a combination of genes: TNFRSF1B, TNFAIP6, HAPLN1 and HAS2, all of them connected to plasticity. When a surrogate value representing the combined expression of said four genes is higher than a reference value, the subject is at risk of suffering metastasis or is not responding adequately to the administered chemotherapy. The method allows the identification of such subjects very accurately, making possible changing the chemotherapy or taking other measures to control cancer progression and/or metastasis generation, thus improving the survival probability of the subject.

## Description

### FIELD OF THE INVENTION

The present invention relates to the field of prognosis of cancer risk and evolution. More specifically, the invention relates to method for the identification of subjects with increased risk of suffering of metastasis and/or being bad responders to chemotherapy, by the determination of the expression level of a combination of genes.

### BACKGROUND OF THE INVENTION

Cancer is a genetic disease. For this reason, most research is still focused on alterations in cancer cells. However, cancer cells grow in close interaction with their microenvironment. The stromal response to tumour cells (e.g., immune cell infiltration, angiogenesis and generation of extracellular matrix) is part of the host defence program, but unfortunately several aspects of this stromal reprogramming support tumour progression and metastasis. Furthermore, there is increasing evidence that the microenvironment substantially contributes to resistance against chemotherapy in metastatic disease. This bi-directional interaction allows cancer to continuously evolve, complicating its treatment enormously.

This cellular evolution has been classified by the acquisition of different hallmarks. Recently, a new hallmark has been added to this list: plasticity (Hanahan *et al.,* 2022). Unlocking tumour cell plasticity is an irreplaceable event that allows tumour cells to adapt to the ever-changing environment that a tumour has. This plasticity can be intrinsic, by genetic and epigenetic changes occurring in the tumour cell; and extrinsic, induced by the interaction with the stromal compartment. Moreover, plasticity is the mechanism by which many tumour cells are able to metastasize.

Metastasis remains as the first cancer-related death with very few therapeutic options (Avula *et al.,* 2020). It occurs when cancer cells are able to escape from the primary tumour site and colonize distant organs, re-educating the stroma of the metastatic niche . The metastatic process is utterly inefficient because of the selective pressure that metastatic cells suffer (Massagué *et al.,* 2016). Indeed, to achieve metastasis, tumour cells need to acquire beneficial traits to evade multiple barriers in the human body and survive in hostile environments different from the primary organ (Celià-Terrasa *et al.,* 2016). These advantageous traits include cell plasticity, immune evasion, ability to enter and escape dormancy state and the capacity to settle in a different organ, shape the stromal environment and proliferate (Massagué *et al.,* 2016; (Celià-Terrasa et al., 2016).

Abdominal tumours, such as gastric, pancreatic and ovarian cancers, tend to colonize the peritoneal cavity in their metastatic process, generating what is known as peritoneal carcinomatosis. The occurrence of peritoneal carcinomatosis has been shown to significantly decrease overall survival in patients. The mechanisms controlling this peritoneal colonization are often similar for tumours with different origins (Coccolini *et al.,* 2013).

For instance, a significant type of cancer is pancreatic ductal adenocarcinoma (PDAC). It is the most lethal type of cancer. 5-year survival is very low (around 10%). The vast majority of transcriptomic data available is at very early stages of tumour development, where tumours are still surgically removed. Unfortunately, most patients are detected when the disease is already metastatic. PDAC frequently metastasizes into the peritoneum forming peritoneal carcinomatosis, which are so far not treatable effectively. As in many other cancers, metastasis-initiating cells need to acquire beneficial traits including cell plasticity, immune evasion, dormancy state control and organ colonization. These characteristics can be summarized in broad terms into two main processes, epithelial-to-mesenchymal transition (EMT) and stemness.

One of the stromal components mediating regulating tumour cell evolution is the extracellular matrix (ECM), which is in the intracellular space. The ECM is particularly important in tumour development (Eble & Niland, 2019), as it retains all cytokines and growth factors secreted by the surrounding cells, providing the perfect support for cancer cell growth(Cao *et al.,* 2018). It is one of the most important determinants of whether or not tumour cells can spread (Pearce *et al.,* 2018).

The ECM is a very heterogenous component that comprises protein fibers, like collagens; proteoglycans, like versican or aggrecan; and polysaccharides, like hyaluronic acid. ECM is involved in tissue homeostasis and serves as scaffold of all our organs (Joyce & Pollard, 2009; Walker *et al.,* 2018). In addition, 3D organization of the ECM is important for the proper organ function and interaction between different cellular components (Eble & Niland, 2019). This interplay between cell types and ECM is tightly regulated under physiological conditions, by ensuring cell-cell and cell-matrix adhesion through integrins and E-cadherin (Joyce & Pollard, 2009). However, during tumorigenesis, the cellular signalling network is disrupted, resulting in aberrant remodelling of the ECM (Calvo *et al.,* 2013; Apte *et al.,* 2013). During this remodelling, the ECM is actively being synthesized and degraded, a process that consumes great deal of energy in fuelling this process (Vennin *et al.,* 2018; Ecker *et al.,* 2019), which gives an idea of how important this process must be.

One of the compounds which is present in the ECM is hyaluronic acid (HA). HA is classified according to its molecular weight in Low (LMW, <500kDa) or High (HMW, >500kDa). HA is synthesized by a family of HA Synthases (HASs) (Walker *et al.,* 2018; Apte *et al.,* 2013). In mammals, there are three different HASs which are tissue and cell-type specific and present different enzymatic activity specially regarding the HA size (Walker *et al.,* 2018; Apte *et al.,* 2013). Hence, HAS1 and HAS2 are involved in HMW-HA synthesis whereas HAS3 is in charge of the LMW-HA formation (Walker *et al.,* 2018). LMW-HA promotes proinflammatory signals contrary to HMW-HA, which promotes antiangiogenic and anti-inflammatory signals (Walker *et al.,* 2018; Eckerd *et al.,* 2019; Spicer *et al.,* 2003). In keeping with this, the group of the present inventors demonstrated that, in ovarian cancer, HMW-HA is responsible of the immunosuppressive role of tumour associated-macrophages to promote the peritoneal spread of tumour cells (Goosens *et al.,* 2019).

Several HA receptors have been identified: CD44, RHAMM, LYVE-1 and ICAM-1 (Vennin *et al.,* 2018; Spicer *et al.,* 2003). Moreover, the group of the present inventors also found recently that endothelial cells, through Notch-dependent CXCL2 expression, preconditions monocyte-derived macrophages to overreact to HA by increasing the availability of one of its receptors, CD44 (Alsina-Sanchis *et al.,* 2022). HA is also known to be a crucial factor in regulating EMT and sternness in PDAC. Hence, it is reasonable to speculate that interfering with HA signalling could have a benefit impact in the treatment of peritoneal carcinomatosis; however, HA is so far not successfully targetable.

As commented above, the interplay between cell types and ECM is tightly regulated under physiological conditions, by ensuring cell-cell and cell-matrix adhesion through integrins and E-cadherin (Joyce & Pollard, 2009). HA is crosslinked into the ECM and its function depends on the molecules that interact with it. Tumour necrosis factor-inducible protein 6 (TNFAIP6; also known as: TSG-6) is a small protein (35kDa) composed of two domains: one binds HA and alter its conformation, the other one mediate dimerization of the protein itself. This causes crosslinking of individual HA molecules and has a dramatic effect on HA structure, collapsing and stiffening HA network (Baranova *et al.,* 2011; Banerji *et al.,* 2007; Richter *et al.,* 2018). During the ovulation decreases the interaction of TNFAIP6 with HA and increases the formation of HC-HA (Baranova *et al.,* 2013; Baranova *et al.,* 2014) leading to a much more hydrated and expanded HA network compared to complexes of TNFAIP6 and HA. This suggests a tight regulation in the ovary, which could be involved also in tumour development.

Hyaluronan and proteoglycan link protein 1 (HAPLN1) is a 40 kDa protein constituted by an immunoglobulin module and two consecutive link modules (similar to those in TNFAIP6). HAPLN1 was first discovered in cartilage where it stabilizes the noncovalent binding of aggrecan to hyaluronan, giving cartilage its load bearing property. The immunoglobulin module mediates the interaction between HAPLN1 and aggrecan (Grover & Roughley, 1994; Matsumoto *et al.,* 2003). In contrast, HAPLN1 and versican G1 interaction occurred via their link modules (Matsumoto *et al.,* 2003). They do not bind directly to each other in solution, but they form ternary complexes with hyaluronan oligomer (Seyfried *et al.,* 2005). Those studies indicate that there is considerable complexity in the interaction of HAPLN1 and chondroitin sulfate proteoglycans (CSPGs), and this may lead to a big variety of quaternary structure of the ternary complex (CSPG-HAPLN1-HA) (Oohashi, *et al.,* 2015).

The group of the authors of the present invention, working with an animal model of PDAC-induced peritoneal carcinomatosis, observed a difference in the immune population within the peritoneal cavity when HPLN1 was expressed in tumour cells. It appears that the increase in HMW-HA induced by HAPLN1 modified the immune microenvironment, as many less neutrophils and monocytes were recruited, while the number of tumour associated macrophages (TMAs) present increased (Wiedmann *et al.,* 2023). In that same publication, HAPLN1 is presented as a prognosis marker and an inductor/operator which cooperates in the generation of peritoneal metastases in PDAC, because it increases tumour cell plasticity and metastasis, its expression is increased in the basal subtype of PDAC and its expression increase is associated with acceleration of peritoneal dissemination of tumour cells.

HAPLN1 has been proposed as a target for the prevention or reduction of metastasis. Thus, for instance, in international patent application WO2020072640A1, and contrary to what would be expectable from the results reported in the previously mentioned article (Wiedmann et al., 2023), it is suggested to increase HAPLN1 expression level or to administer compositions comprising HAPLN1 with the aim of preventing or reducing metastasis risk. The description of WO2020072640A1 is focused mainly in melanoma and lymphatic system, although it also relates to pancreatic and ovarian cancer and the prevention of metastases originated from them.

In other documents, such as WO2020232033A1, HAPLN1 is mentioned as one of the genes that can be used to classify or predict the status of several cancers from two or more predictions. Pancreatic or ovarian cancers are not explicitly mentioned.

In other publications it is also possible to find some contradictory results regarding genes linked to HA and their influence in cancer prognosis and survival. Riecks *et al.,* (Riecks *et al.*, 2022) analysed the influence of hyaluronane synthases (HAS1-3) and hyaluronidases (HYAL1-4, PH20 and HYALP1) in survival of patients suffering from ovarian cancer, finding that the expression of HAS2, HYAL2 and HYAL3 is downregulated in ovarian cancer related to the control. However, low expression of HAS2 is said to favoured patient survival.

Other authors (Nie *et al.,* 2021) have proposed prognostic signatures for ovarian cancers, based specifically on 14 genes that are considered to be tumour microenvironment-related genes, which are suitable for predicting metastasis risk. But, in this case, neither HAS2 or HAPLN1 are among the proposed genes, which are: ELN, FBLN1, ANGPT2, FBL, COMP, PPL, PLD2, PDLIM4, EMP1, MYC, ITGAM, FRAT2, WDR45, and ADSL.

As can be seen, the identification of the mechanisms favouring metastasis, the influence of HA and proteins which interact with said compound and the search for a way of identifying patients in risk of developing metastasis are important fields of research, especially in the case of abdominal tumours such as pancreatic and ovarian cancers, but the results are not always clarifying enough for designing robust methods for the identification of subjects in risk of developing metastasis, and they are sometimes a bit contradictory, so that one skilled in the art, having to study the risk of developing metastasis of an ovarian cancer patient, for instance, would not be very sure about how to interpret the implications for metastasis development and survival of high levels of genes such as HAPLN1 or how to design a reliable method for identification of patients suffering or having suffered an abdominal cancer and being in risk of developing metastasis.

The high number of PDAC patients that exhibit metastases when their demise occurs highlights the importance of understanding mechanisms underlying peritoneal metastasis, particularly those stemming from PDAC or ovarian tumours. It would be also very important to have a method of identifying those patients that are prone to develop metastases and/or those with a high probability of not responding to the chemotherapy treatment currently used. Preferably, the method should be reliable and able to identify subjects in risk of developing metastasis easily. Also preferably, the method should provide the possibility of identifying patients that would not respond to the current chemotherapeutic methods used for pancreatic and/or ovarian cancers, so that they can benefit from an alternative chemotherapy that really helps to control their risk of cancer worsening and development of metastasis.

The present invention provides a solution to such problem.

### SUMMARY OF THE INVENTION

The present invention is based on the finding that a gene signature based on the expression of the genes TNFRSF1B, TNFAIP6, HAPLN1 and HAS2, considered in combination, has predictor capacity to identify those patients with high risk of suffering metastasis from abdominal cancers as well as to identify those patients that would be bad responders to the chemotherapeutic methods currently applied against pancreatic and/or ovarian cancer.

The authors of the present invention have found that such signature has a strong correlation to patient survival, stronger than that of HAPLN 1 expression alone and also stronger than that of TNFRSF1B, TNFAIP6 or HAS2 expression considered separately. The correlation is even stronger than the correlation corresponding to the combination of the genes TNFAIP6, HAPLN1 and HAS2, which has a predictor capacity that is improved with the inclusion of the gene TNFRSF1B in the signature. Besides, it is a signature that clearly defines those patients that are almost certainly going to develop metastasis, being thus very reliable. And, moreover, the signature also allows to predict bad responses to chemotherapy, since it has been verified that patients with higher expression of the genes forming the signature respond worse to chemotherapy. The signature is valid for abdominal cancers, such as pancreatic (especially pancreatic ductal abdominal cancer) and ovarian cancer.

As detailed in the Examples section, the invention arises from the clarification of the role of HAPLN1 in abdominal cancers, particularly pancreatic and ovarian cancer, confirming that HAPLN1 high expression is correlated with shorter overall survival and with an enrichment in EMT signatures. Among others, HAPLN1 significantly increases TNF-α dependent expression of HAS2, TNF-α induced invasion being dependent on HA synthesis.

Additionally, HAPLN1 also induced the expression of TNFRSF1B (tumour necrosis factor receptor superfamily member 1B, also known as TNFR2: tumour necrosis factor receptor 2), which leads to the upregulation of HAS2 and TNFAIP6. Survival analysis showed that, surprisingly, the contribution of all of these genes, together, is higher than the contribution of any of each gene separately, being useful as a signature for metastasis risk prognosis and chemotherapy response prediction.

Thus, the present invention relates to a method for determining the risk of developing metastasis, the survival and/or the response to the administered chemotherapy in a subject under study who suffers or has suffered from an abdominal cancer, comprising the steps of:
a) determining an expression value for each one of the genes of the group of TNFRSF1B, TNFAIP6, HAPLN1 and HAS2 in a tumour sample of the subject,
b) obtaining, by multivariate analysis, a first value which represents the expression value of a surrogate gene that represents the combined expression of aforementioned four genes in the subject;
c) obtaining, also by multivariate analysis, a second value which represents the mean expression value of the surrogate gene that represents the combined mean expression of the aforementioned four genes in a population of subjects suffering from the same cancer,
d) comparing the value obtained in b) with the value obtained in c),
e) concluding that the subject under study is:
   i) at risk of developing metastases and/or is not responding to the administered chemotherapy if the value obtained in b) for the subject is higher than the value obtained in c) for the population of cancer patients, and/or
   ii) at low risk of developing metastases or in a high probability of not developing metastases, and/or responding to the administered chemotherapy if the value obtained in b) for the subject is equal or lower than the value obtained in c) for the population of cancer patients.

Preferably, the mean expression value of the surrogate gene is obtained performing Surrogate Variable Analysis (SVA) with the (mean) expression value of each gene of the group of TNFRSF1B, TNFAIP6, HAPLN1 and HAS2, adjusting for unmeasured or unknown sources of variation in the data. Also preferably, for the analysis of the sample(s) taken from the subject, the expression value of each gene of the group of TNFRSF1B, TNFAIP6, HAPLN1 and HAS2 is the mean value of several data obtained either from one or several samples of the same subject. Also preferably, the tumour sample is a biopsy. Regarding the population of cancer patients, the expression value of each one of the aforementioned four genes will be the mean value of the expression values obtained for that gene for each one of the cancer patients of the population, while the mean expression value of the surrogate gene that represents the expression of all four genes will be calculated by the same type of multivariate analysis, preferably SVA also adjusting for unmeasured or unknown sources of variation in the data.

Preferably the level of expression of each of the genes TNFRSF1B, TNFAIP6, HAPLN1 and HAS2 is determined by analysing the level of the messenger RNA and/or the level of the protein expressed by each one of the genes.

It is preferred that the abdominal cancer is ovarian cancer or pancreatic cancer, pancreatic ductal abdominal cancer being particularly preferred. The cancer can also be oesophageal, liver or cervical cancer. With regard to the subject, it will be preferably a human being, although the invention can be also applied to other mammals, such as dogs, cats, rabbits, cows and bulls, horses, donkeys, or any others.

The method of the present invention, based on four genes connected with plasticity acquisition, means a reliable procedure for the identification of those subjects suffering (or having suffered) from an abdominal cancer at risk of developing metastasis, what makes easier to treat the subject before the metastasis dissemination starts or when it has just started and applying a treatment in order to control metastasis spreading as soon as possible. As the method also allows to detect those patients undergoing chemotherapy that are not responding to it, it makes possible to change the therapeutic treatment and to replace it for another one that can be more effective for such subject.

The invention will be now explained with more detailed in the following sections.

### BRIEF DESCRIPTION OF THE DRAWINGS

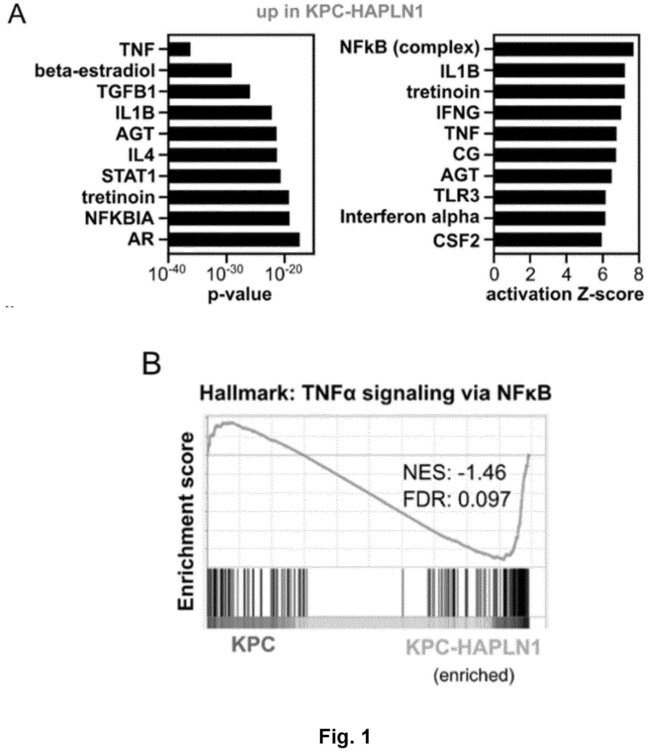

Fig. 1 HAPLN1 potentiates TNFα actions. A. Top 10 upstream regulators assessed by ingenuity pathway analysis (IPA) of sorted tumour cells from mouse tumours sorted by p value or activation Z score. B. GSEA (Gene Set Enrichment Analysis) of "HALLMARK: TNFα signalling via NFκB" on sorted tumour cells.

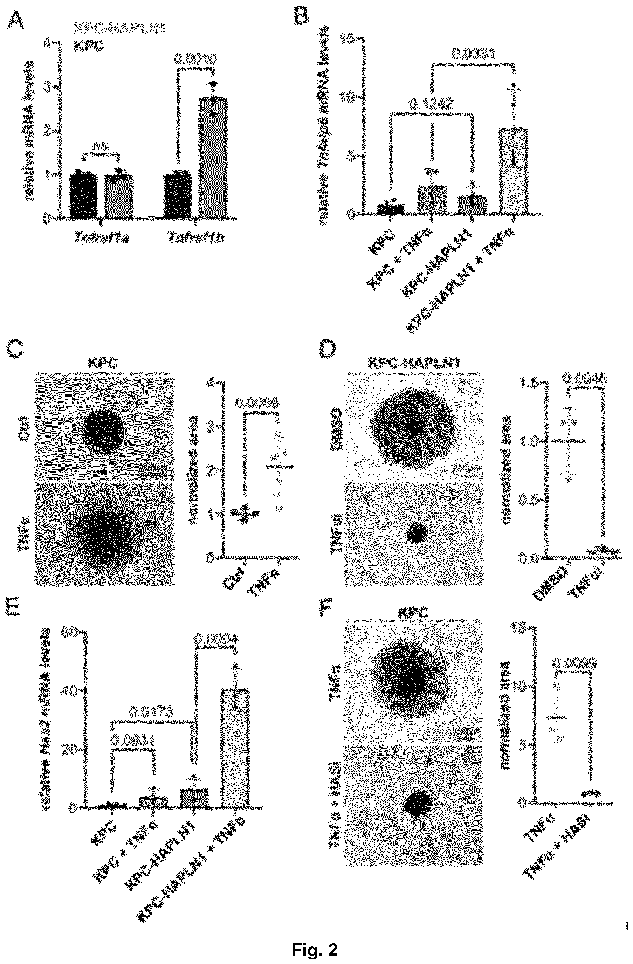

Fig. 2: Increased TNF signalling induces tumour cell plasticity. A. TNFα receptors (*Tnfrsf1a* and *Tnfrsf1b*, first and second pairs of bars, respectively) expression, analysed by qPCR in KPC cells (blue in the original, first bar of each pair) and KPC-HAPLN1 (orange in the original, second bar of each pair) cells , and indicated in relation to the control values, the values obtained in KPC cells. B. Analysis of TNF-induced *Tnfaip6* gene expression in vitro, related to the control value (expression value in KPC cells not incubated with TNFα); n=4. C. Photographs (scale bar: 200 µm) and plot of the normalized area corresponding to the invasion assay into Matrigel of KPC cells stimulated (lower photograph, labelled TNFα) or not (upper photograph, labelled "Ctrl", corresponding to the control) with 50 ng/ml TNFα; invasion was measured as relative occupied area 48 h after embedding, and represented, for each of the 5 replicates for condition (Ctrl or TNFα), using the normalized area calculated in relation to the mean value obtained from the 5 control samples. D. Invasion assay of KPC-HAPLN1 cells into Matrigel using a TNFα antagonist (10 µg/ml) (photographs and data labelled "TNFαi") or using DMSO as control treatment (photographs and data labelled "TNFαi"); the values plotted on the right side of the panel were calculated like in panel C, for n=3. E. Relative mRNA levels of Has2 after 6 h of 50 ng/ml TNFα stimulation in KPC (first pair of bars) and KPC-HAPLN1 (second pair of bars); the relative values were calculated with reference to the mean value obtained in non-stimulated KPC cells (n=3-4). F. Invasion assay of KPC cells into Matrigel after addition of 50 ng/ml TNFα alone (upper photograph and first line of values of the plot, all labelled "TNFα"), or with 500 µM 4-methylumbelliferrone, HASi (lower photograph and second line of values of the plot, all of them labelled "TNFα + HASi"); pictures taken 48 h after embedding and stimulation; the values plotted on the graph of the right side of the panel were calculated like in panel C (mean+SD), for n=3; scale bar: 100 µm. As commented above, graphs represent mean±SD. Data points indicate independent biological replicates. Unpaired two-tailed T test was used for statistical analysis.

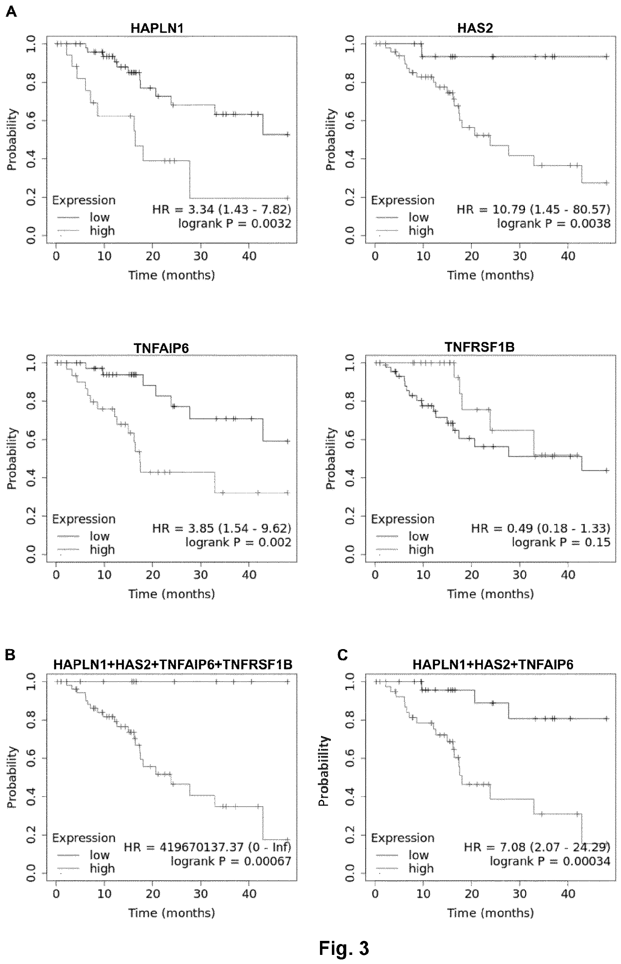

Fig. 3: Metastasis prognosis potential in PDAC: relapse free survival curves using, HAPLN1, HAS2, TNFAIP6 and TNFRSF1B expression as a classifier; Alone (A), combining all four genes (B) or combining only HAPLN1+HAS2+ TNFAIP6 (C).

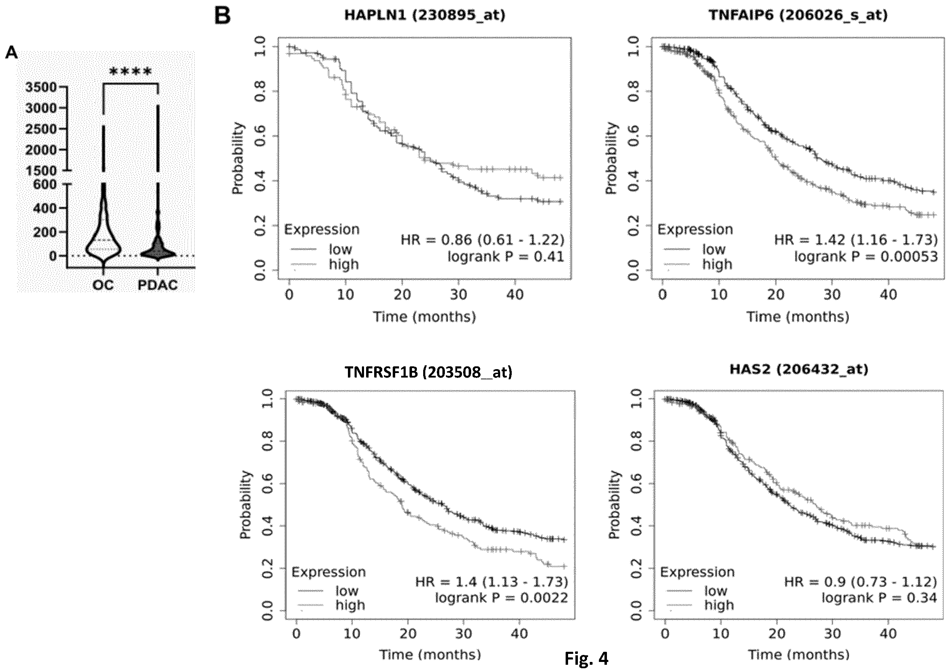

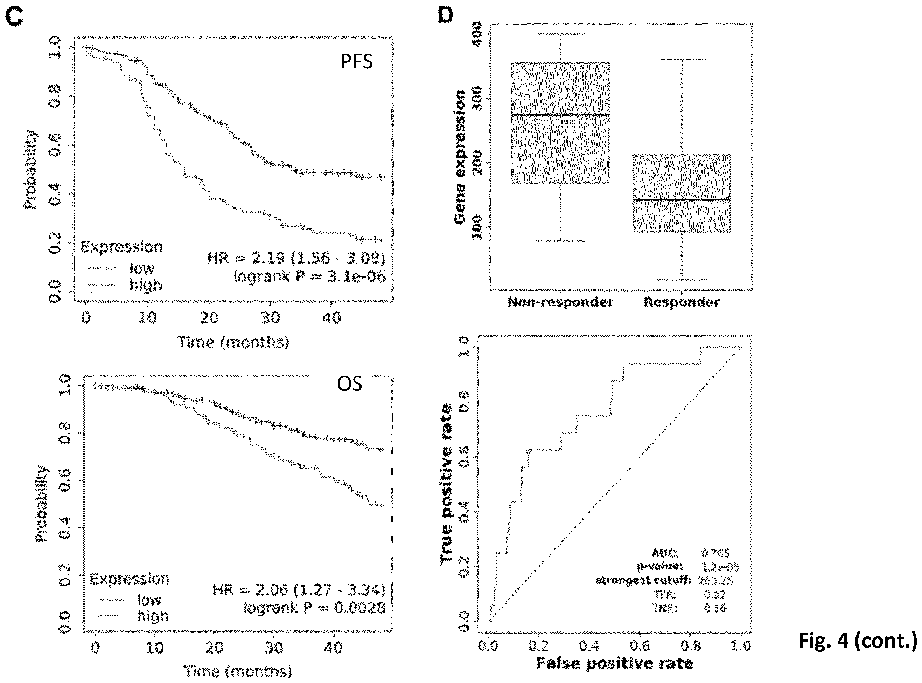

Fig. 4: HAPLN1 expression in ovarian cancer is higher than in PDAC and correlates with worse prognosis. A: HAPLN1 expression levels in patients suffering from ovarian cancer (OC) or pancreatic ductal adenocarcinoma (PDAC) from the TCGA (The Cancer Genome Atlas) database. B. Progression free survival curves using each gene individually HAPLN1 (probe ID: 230895_at), TNFAIP6 (probe ID: 206026_s_at), TNFRSF1B (probe ID: 203508_at) and HAS2 (probe ID: 206432_at) expression as a classifier; as indicated over each graph, where the code of the Affimetrix^{™} array probe used for its detection is also indicated). C. Survival curves (PFS: progression free survival; OS: overall survival) using TNFAIP6, HAPLN1, HAS2 and TNFRSF1B combined expression as a classifier. D. Receiver operating characteristic curve, ROC, plot based on TNFAIP6, HAPLN1, HAS2 and TNFRSF1B expression: the upper graph depicts differential expression between responders and non-responders; the lower one represents the area under the curve (AUC: 0.765) for the response of the two classes of patients, where the values corresponding to the p-value, the strongest cutoff, TPR (true positive rate) and TNR (true negative rate) are also indicated.

### DETAILED DESCRIPTION OF THE INVENTION

As described above, the present invention relates to the identification of those subjects being in risk of developing metastasis, whose survival time free of metastasis is lower than 100 months and/or who are not responding to the administered chemotherapy, which identification is carried out from the expression level of a signature of genes consisting of TNFRSF1B, TNFAIP6, HAPLN1 and HAS2. Specifically, said genes are as follows:
- TNFRSF1B (tumour necrosis factor receptor superfamily member 1B, also known as TNFR2: tumour necrosis factor receptor 2, and also CD120b), (Human gene and protein data: HGNC ID:11917, ENSEMBL: ENSG00000028137, NCBI Gene 7133 (updated on 27-August-2023), NCBI RefSeq NM_001066.3, UniProt/Swiss-Prot: P20333; Mouse gene and protein: MGI:1314883; ENSEMBL: ENSMUSG00000028599, NCBI Gene: 21938, updated on 18 August 2023, NCBI Reference Sequence: NM_011610.3, UniProt/Swiss-Prot:P25119);
- TNFAIP6 (TNF alpha induced protein 6, also known as TSG-6 or TSG6) (human gene and protein data: HGNC ID:11898, Ensembl:ENSG00000123610, NCBI Gene 7130 (updated on 14-August-2023), NCBI RefSeq NM_007115.4, UniProt/Swiss-Prot: P98066.2; Mouse gene and protein: MGI:1195266; Ensembl: ENSMUSG00000053475, NCBI Gene: 21930, updated on 05 August 2023, NCBI Reference Sequence: NM_009398.2, U n i Prot/Swiss-Prot: 008859.1);
- HAPLN1 (hyaluronan and proteoglycan link protein 1, also known as CRT1 and CRTL1) (human gene and protein data: HGNC ID:2380, ENSEMBL: ENSG00000145681, NCBI Gene 1404 (updated on 6 September 2023), NCBI RefSeq NM_001884.4, UniProt/Swiss-Prot: Q9819.1; Mouse gene and protein: MGI:1337006; Ensembl: ENSMUSG00000021613, NCBI Gene: 12950, updated on 06 September 2023, NCBI Reference Sequence: NM_013500.4, UniProt/Swiss-Prot: Q9QUP5.1);
   and
- HAS2 (hyaluronan synthase 2) (human gene and protein data: HGNC ID:4819, ENSEMBL: ENSG00000170961, NCBI Gene 3037(updated on 3 September 2023), NCBI RefSeq NM_005328.3, UniProt/Swiss-Prot: Q92819.1; Mouse gene and protein: MGI:107821; Ensembl: ENSMUSG00000022367, NCBI Gene: 15117, updated on 05 September 2023, NCBI Reference Sequence: NM_008216.3, UniProt/Swiss-Prot: P70312.4).

When they are considered in combination, the signature that combines all of them has predictor capacity to identify those patients with high risk of suffering metastasis from abdominal cancers as well as to identify those patients that would be bad responders to the chemotherapeutic treatment that the patient is receiving.

In order to analyse together the expression values of all four genes, taken together, as a combination, a substitute value (the value of a surrogate variable) is calculated that represents the whole signature of four genes, facilitating the handling and comparison of data. Surrogate variables are covariates constructed directly from high-dimensional data (like gene expression/RNA sequencing/methylation/brain imaging data) that can be used in subsequent analyses to adjust for unknown, unmodeled, or latent sources of noise. For the purposes of the present invention, the surrogate variable is treated as it were the expression value of a surrogate gene. Thus, in the present invention, the value considered to be the expression value of the surrogate gene represents the combined expression value of all four genes and acts as a substitute of the combination of all four genes. The expression of the surrogate gene is calculated by multivariate analysis, a technique which has as its aim to find patterns and correlations between several variables simultaneously. Preferably, for the present invention, surrogate variable analysis (SVA) is used (for instance, using the Kaplan-Meier plotter tool, accessible through the web site https://www.kmplot.com, as in Example 2 of the present application), which allows to calculate a substitute value (the expression value of the surrogate gene) of the expression of all four genes of the signature by means of an algorithm which allows modelling the effect expression heterogeneity (EH) in gene expression matrices. The EH is used to describe the patterns of variations due to the influence of any variable not taken into account in the model.

The comparison of the expression value of the surrogate gene obtained of the subject under study and the expression value of the surrogate gene obtained from a population of patients suffering from the same type of cancer allows to reach conclusions about the risk of the subject under study of developing metastases and/or his/her response to the chemotherapy that such subject is receiving. Thus, the value obtained from the population of cancer patients acts as a reference value which facilitates to extract conclusions about the risk of developing metastases and/or the response to chemotherapy for a specific subject.

The present invention is based on conclusions extracted from the assays shown in the Example section, in which section assays can be found that shown that the mentioned signature of four genes has a strong correlation with patient survival, stronger than the correlation obtained when expression of any of the four genes is taken separately.

In the particular case of pancreatic ductal adenocarcinoma, it can be seen that the probability of relapse free survival is 1 for more than 2 years (using a time limit of 48 months) for the subjects having an expression of the surrogate gene of all four genes equal or lower than the expression value of the surrogate gene obtained from the selected population of cancer patients. Such probability value shows the strength of the correlation. This is because none of the patients with low expression values of the signature reported a relapse in the following 48 months after resection. When the same analysis is made for each gene isolated, the hazard ratio for relapse is lower with all the genes, even when the best of the predictor genes is considered, in this case HAS2. This indicates that the contribution of these genes is giving an extra prediction value that is not explained by an additive effect of each of them. This is particularly clear when the graph of Fig. 3B is compared, for instance, with the graph of Fig. C, which allows to see that the simply introduction of an additional gene, TNFRSF1B, in a signature that already contains three genes, improves the prediction capability of the method, making clearer the difference between patients with low probability (exactly, no probability during 48 months) of relapsing and those with high probability. Introducing TNFRSF1B in the signature also increases clearly the hazard ratio. And said results are achieved, surprisingly, after including in the signature a gene which gives rise, individually, to results about the predicted evolution of the patients different from those obtained with the other genes of the signature: for TNFRSF1B, patients with high expression of the gene show a probability of relapse free survival better (higher) than patients with low expression at least until month 30 is passed, when the probabilities for ones and other patients are practically equal during 10 additional months, contrary to what happens with any of the other three genes of the signature considered individually. The pattern observed for TNFRSF1B individually makes surprising the results obtained with the whole signature of four genes which comprises TNFRSF1B as a member of the signature.

When the whole signature of the four genes is considered, the high risk of suffering metastases is clearly closely linked to the survival time free of metastasis and, additionally, tumour regression and metastasis appearance are also linked to the overall survival time. That is the reason why the method can be also envisaged as a method for the prognosis of the subject survival time, either as the survival free of relapse or as the overall survival time.

As can be seen in Fig. 4, the hazard ratio found in survival analysis of ovarian cancer is higher than 2, (2.19 in the case of the progression free survival and 2.06 in the overall survival analysis). Accordingly, subjects who have suffered from ovarian cancer and show expression values of the surrogate gene representing all the four genes of the signature equal or lower than the expression value of the surrogate gene calculated for the selected population of cancer patients have a probability of cancer progression-free survival and overall survival more than 2 times higher than a subject with at least one of the genes with an expression value higher than that of the reference value for that gene. Additionally, subjects who have suffered from ovarian cancer and show expression values of the surrogate gene representing all the four genes of the signature equal or lower than the expression value of the surrogate gene obtained from the selected population of cancer patients have a probability of more than 40% of cancer progression-free survival during at least 48 months; their probability of overall survival is more than 70% considering a term of 48 months. However, subjects who have suffered from ovarian cancer and show expression values of the surrogate gene higher than those calculated for the population of cancer patients have a probability of approximately 20% of cancer progression-free survival during at least 48 months; their probability of overall survival is only a bit higher than 40% during at least 48 months.

In the particular case of pancreatic cancer, specifically PDAC, the probability of survival free of progression (relapse) (RFS) is similar than that of the subjects having suffered from ovarian cancer for a term of 48 months, for the subjects showing high level of expression of the surrogate gene representing the 4 genes of the signature, being scarce the cases which exceed the 40-42 months of relapse free survival. The hazard ratio found in the relapse free survival of PDAC is high, higher than 4×10⁸, and the probability of remaining free of relapse is 100% during 48 months after tumour resection for those subjects having expression values of the surrogate gene lower than the value obtained from the population of cancer patients . Accordingly, subjects who have suffered from PDAC and show expression values of the surrogate gene representing the signature equal or lower than the surrogate value obtained from the population of cancer patients have a probability of cancer progression-free survival more than 4×10⁸ times higher than a subject with an expression value of the surrogate gene higher than that of the expression value of the surrogate gene obtained from the population of cancer patients . Additionally, subjects who have suffered from PDAC and show expression values of the surrogate gene representing all the four genes of the signature equal or lower than the reference value for that signature of genes (the expression value of the surrogate gene resulting from the population of cancer patients) have a probability of 100% of cancer progression-free survival during at least 48 months. However, subjects who have suffered from PDAC and show expression values of the surrogate gene representing the four genes of the signature higher than the reference value (the expression value of the surrogate gene resulting from the population of cancer patients) have a probability higher than 80% of cancer progression-free survival only during approximately 10 months, which lowers progressively until approximately 40-42 months: at a value higher than 50% after 20 months, approximately 40% after 30 40 months, being of only 20% after approximately 42 months.

Therefore, in the step d) of making conclusions of the method of the present invention, it can be considered that the subjects exhibiting, for the surrogate gene representing the signature, a lower expression value than the surrogate gene representing the signature for the cancer patient population (the reference value), have almost no probability of relapse and therefore have a much better prognostic than their counterparts. In the specific case of PDAC, when the surrogate gene representing the genes of the signature shows high levels of expression, it can be concluded that relapse is absent in at least 48 months after tumour resection. While in ovarian cancer patients the probability of relapse is more than twice higher in those subjects showing higher expression of the surrogate gene representing the signature (that is, the probability of cancer progression free survival is more than twice higher in those subjects with lower expression of the surrogate gene).

With regard to chemotherapy, usually, the chemotherapy that the subject will be receiving will be, probably, the one currently most used for said cancers. Thus, for pancreatic cancer, it will be possibly gemcitabine or a cocktail of several drugs such as: folfirinox, gemcitabine and capecitabine, or gemcitabine with paclitaxel. In the case of ovarian cancer, the most commonly drug used after surgery is carboplatin, sometimes combined with paclitaxel. Irrespective of the administered chemotherapy, a high value of expression of the surrogate gene representing all the four genes of the signature (higher than the expression value obtained for the population of cancer patients) will indicate that the chemotherapy is not functioning as desired and the subject is in risk of progression of the tumour and developing metastasis, so that it would be advisable to change it.

The method is applicable to any mammal. Usually, the subject will be a human being (which is one of the preferred embodiments of the present invention, combinable with any other one), but it can also be a domestic animal (dogs, cats, hamsters...) or an animal used for food production, as work force or used for leisure (for instance, horses and mares, donkeys, bulls, oxen and cows, pigs, rabbits, etc.). More specifically, the invention is particularly useful for those subjects that are suspected to being suffering from an abdominal cancer or, more frequently, who have been the subject of surgery to resect an abdominal tumour, because tumours are not always completely resected and a risk of tumour progression and/or metastasis remains, even when chemotherapy is being administered. The method of the present invention will be useful to assess such risk and/or if the subject is responding to administered chemotherapeutic treatment.

The biological sample taken from the subject under study (the subject where it is desired to assess the risk of metastasis and/or the response to chemotherapy) will be a sample of a biopsied tumour.

The expression of each one of the four genes of the signature can be calculated by determining, for instance, the level of the corresponding messenger RNAs (mRNAs) and/or the concentration of the proteins expressed by the genes. This can be done by any method known for those skilled in the art. For instance, mRNA level can be determined by qRT-PCR, like in Example 1 of the present application, by quantification from a Northern blot or by any other method. Concentration of each one of the proteins encoded by the four genes of the signatures can be determined by mass spectrometry, quantification from a Western blot, affinity chromatography followed by a selected identification and quantification technique, by a methodology linked to the use of antibodies specific for each protein that allows subsequent quantification (such as immunoassays like ELISA) or any other known methodology.

With regard to the reference value (the value considered the mean expression value of a surrogate gene calculated from the expression values of the four genes of the signature in a population of cancer patients), a mean value for subjects of the same species than the subject under study and suffering (or having suffered) from the same particular abdominal cancer under monitorization can be used. Such reference value can be extracted from the data recorded in a database, preferably public and dedicated to cancer, such as The Cancer Genome Atlas (TCGA, accessible from: http://cancergenome.nih.gov/) database, as in Example 2 of the present application, or can be the result of any another study carried out on a selected population of subjects suffering (or having suffered) from the same abdominal cancer where the mean value of the surrogate gene corresponding to the whole population will be used. This last option can be the preferred alternative when the method of the present invention is applied to specific groups of individuals (same race, same gender, same birth or inhabitation area, individuals sharing any other feature...). The samples can be histological samples of cancer patients, like in Example 1, from the biobank present in most hospitals, which keep biopsies of every patient treated. The expression of these four genes can be measured by qPCR and the mean expression of the surrogate gene calculated. This will give a reference value of a populations as similar as it can be to the patient to be diagnosed.

As explained above, the expression value calculated for the surrogate gene representing the combined expression of all four genes of the signature must show values higher than those of the reference value calculated for the selected population of cancer patients in order to consider that the subject under study is in risk of developing metastases and/or is not responding (or not responding well enough) to the administered chemotherapy.

The invention will be now further explained by means of the Examples below.

### EXAMPLES

The assays set forth in the following Examples have been carried out with the following materials and methodologies:
All mouse experiments were approved by the local authorities (RP Karlsruhe and DKFZ) and carried out following their legal requirements. Approval for using the resection samples was obtained from the institutional ethics committee (#24-4-20). All procedures were designed and performed in compliance with the Declaration of Helsinki and all institutional, state and federal guidelines.

### Patient samples

Pancreatic cancer patient histology samples were obtained from the Institute of Pathology of the University Medical Center Goettingen.

### Patient expression data of human epithelial, immune and stromal PDAC cells

Transcriptomic data of PDAC epithelial, immune, and stromal cells were obtained from PDAC specimens of patients who received partial pancreatoduodenectomy at the Department of General, Visceral and Transplantation Surgery, University of Heidelberg. Patients were part of the HIPO-project. The study was approved by the ethical committee of the University of Heidelberg (case number S-206/2011 and EPZBiobank Ethic Vote #301/2001) and conducted in accordance with the Helsinki Declaration; written informed consent was obtained from all patients. Epithelial, immune, and cancer-associated fibroblast populations were isolated via were isolated by fluorescence activated cell sorting using FITC anti-human CD326 (EpCAM, 1:11, Clone: AC128, Miltenyi Biotec) and VioBlue anti-human CD45 (1:11, Clone: 5B1, Miltenyi Biotec) after cell death exclusion using propidium iodide. RNA extraction, library preparation and RNA sequencing were performed as previously described (Cabezas-Wallscheid, N. et al., 2014). Briefly, cDNA libraries were generated with 10 ng of total RNA for HSC-MPP using the SMARTer Ultra Low RNA kit for Illumina sequencing (Clontech Laboratories) according to the manufacturer's indications. Sequencing was performed with a HiSeq2000 device (Illumina) and one sample per lane. Sequenced read fragments were mapped to the mouse reference genome GRCm38 (ENSEMBL release 69) using the Genomic Short-Read Nucleotide Alignment program.

### qRT-PCR

For transcriptomic analysis, cell lines were lysed, and RNA was isolated according to manufacturer's protocol of the innuPREP RNA Mini Kit (Fisher Scientific, 10489573). Bulk tumour samples were homogenized with metal beads and RNA isolated with help of Trizol reagent (Life Technologies, 15596026) and isopropanol precipitation. RNA of sorted cells was isolated using the PicoPure RNA Isolation Kit (Life Technologies, KIT0214). Reverse transcription was performed with the High- Capacity cDNA Reverse Transcription Kit (Life Technologies, 4368814) or the SuperScript IV VILO Master Mix (Life Technologies, 11766050). qRT-PCR was executed using Power SYBR Green PCR Master Mix (Life Technologies, 4368708) and the primers listed in Table 1 below. Relative expression levels were calculated with the 2-ΔΔCt method after normalization to the house keeping gene Hprt.

**Table 1.- Primers for qRT-PCR**

| Gene name | Forward primer 5'-3' | SEQ ID NO: | Reverse primer 5'-3' | SEQ ID NO: |
|---|---|---|---|---|
| HAS2 | CTCTTTTGGACTGTATGGTGCC | 1 | AGGGTAGGTTAGCCTTTTCACA | 2 |
| HPRT | CCTGGCGTCGTGATTAGTGAT | 3 | AGACGTTCAGTCCTGTCCATAA | 4 |
| HAPLN1 | TCTGGTGCTGATTTCAATCTGC | 5 | TGCTTGGATGTGAATAGCTCTG | 6 |
| TNFAlP6 | TTTCTCTTGCTATGGGAAGACAC | 7 | GAGCTTGTATTTGCCAGACCG | 8 |
| TNFRSF1B | TGAAACATCAGACGTGGTGTG | 9 | TGCAAATATCCGTGGATGAAGTC | 10 |

### Cell culture maintenance

All cells used for in vitro culture were cultured in DMEM, low glucose (high glucose for HEK293A + T cells), GlutaMAX Supplement (Life Technologies, 21885108) supplemented with 10% FCS (VWR International, S181B) and 1% Pen/Strep (Life Technologies, 15140122). For 2D experiments, cells were starved for 48 h prior to analysis. Cells were periodically analysed by PCR for mycoplasma contamination (primers: forward: gggagcaaacaggattagatataccct (SEQ ID NO:11); reverse: tcggaccatcatctgtcactctgttaacctc (SEQ ID NO: 12). Authentication was not deemed necessary.

### Flow cytometry and cell sorting

Analysis by flow cytometry was performed on a BD LSR Fortessa, while BD FACS Aria/BD FACS Aria Fusion Sorters were used for cell sorting. The listed antibodies or dyes in Table 2were incubated for 30minwith the cells before washing and analysis/sorting. For intracellular staining, cells were first stained with extracellular staining, then fixed, permeabilized and intracellular staining for 20min was performed. Cell cycle was analysed in fixed cell (70% ethanol) by permeabilization and DNA staining with propidium iodide (5 µg/mL, Sigma-Aldrich, 537059) in presence of RNase A (1mg/mL, Sigma-Aldrich, R5125). Compensation was carried out with UltraComp eBeads Compensation Beads (Life Technologies, 1-2222-42). FlowJo Software was used for analysis of the acquired samples.

### Generation of (stable) cell lines

Stable overexpression was achieved by lentiviral infection of KPC and PANC-1 cells (ATCC). For HAPLN1 overexpression the sequence coding for HAPLN1 was inserted into a pLenti6-V5dest plasmid. The plasmid pLL3.7 (Addgene #11795) was used for eGFP, while EF.CMV.RFP plasmid (Addgene #17619) or pLenti-RFP-Puro (Hölzel, LTV-403) was used for RFP labelling. The plasmid of interest was transfected together with the plasmids for packaging (psPAX2) and envelope (pMD2, encoding for VSV-G) into HEK293T cells. Cells were infected and successful integration into the genome was guaranteed by selection with Blasticidin-Hydrochloride (Sigma Aldrich, 15205) for a minimum of 10 days. For control cells, an empty pLenti6-V5dest plasmid was used. eGFP and RFP positive cells were sorted out to receive a homogeneously marked population. For adenoviral infection, plasmids encoding for eGFP and mCherry were amplified in HEK293A cells and cells of interest were infected afterwards. Analysis was carried out no later than 96 h post infection to prevent loss of overexpression.

### Spheroid formation and invasion assay

For 3D culture, 5.000 cells were seeded in a 96 well ultra-low attachment plate (NEO Lab, 174929) in DMEM + 10% FCS. After 96 h, pictures of the formed spheroids were taken with a Zeiss Cell Observer. For analysis by mRNA or flow cytometer, cells were seeded in 6 well plates coated with Poly(2-hydroxyethyl methacrylate) (Poly-HEMA, Sigma Aldrich, P3932) and afterwards lysed, or digested with 0,05% Trypsin (Life Technologies, 25300054) for 5 min respectively. Subsequential to digestion, cells were stained with DAPI for analysis by flow cytometry. For invasion assay, spheroids were formed for 48 h in 96 well plates, before half of the medium was replaced with Matrigel (Fisher Scientific, 11523550) or Collagen extracted from rat tails. When stimulating the spheroids, they were treated with HA (mix of LMW and HMW HA, both at 5 µg/ml; R&D Systems, GLR001 and GLR002), recombinant HAPLN1 (80 ng/ml, BioTechne, 2608-HP-025), Hyaluronidase (200mg/ml, MP Biomedicals, 151272), recombinant TNFα (50 ng/ml; PeproTech, murine: 315-01A-20, human: 300-01A-50), IgG control antibody (1 µg/ml. Biozol Diagnostica, BXC-BE0090-1MG), anti-CD44 blocking antibody (1 µg/ml. Biozol Diagnostica, BXC-BE0039-1MG), or TNFα inhibitor (10 µg/ml, Merck, 654256), immediately before the addition of matrigel or collagen. When using the following inhibitors spheroids were pre-treated for 24 h with (HASi (4-Methylumbelliferrone (4-MU), 500 µM, Sigma Aldrich, M1381), FAKi (GSK2256098, 10 µM, Cayman, Cay22955-1), STAT3i (Stattic, 25 µM, Abcam, ab120952), PI3Ki (LY294002, 10 µM, BioTrend, HY-10108), MEKi (PD98059, 50 µM, New England Biolabs, 9900 L), ERKi (ERK Activation Inhibitor Peptide I, 1 µM, Sigma Aldrich, 328000) or DMSO as control, before embedding. For cocultures, equal amounts of KPC and KPCHAPLN1 cells expressing either RFP/mCherry or GFP were seeded. 48-120 h after addition of Matrigel or collagen, pictures of invasion were taken by a Zeiss Cell Observer or a Zeiss LSM710 ConfoCor3.

### RNA sequencing

Libraries and multiplexes were prepared with NEBNext Multiplex Oligos for Illumina (New England Biolabs, E7600S) and NEBNext Single Cell/Low Input RNA Library Prep Kit for Illumina (NewEngland Biolabs, E6420S). NextSeq 550 PE 75 HO (Illumina) RNA sequencing was performed by the Genomics & Proteomics Core Facility of the German Cancer Research Center (DKFZ, Germany).

### Classification in HAPLN1high and HAPLN1low cohorts for survival, GSEA and cellular content

For the survival data regarding PDAC the present inventors made use of the publicly available transcriptomic, proteomic and clinical data published in Cao et al. (2021). For the distribution of patients into the HAPLN1high or HAPLN1low cohorts, the group of the present inventors extracted the HAPLN1 expression (obtained by RNA sequencing) or HAPLN1 protein levels (obtained by mass spectrometry) and calculated the mean expression levels over all patients. Patients were assigned to the HAPLN1high cohort, if their HAPLN1 expression level was above this mean, and vice versa. mRNA and protein analyses were conducted independently. Then patients were ordered according to follow up days and vital status, and plotted resulting survival curves. Same classification was employed for the analysis of the cellular content of the tumours, using the clinical data provided by the authors of the dataset.

### Gene Set Enrichment Analysis (GSEA)

To investigate differentially expressed genes, Gene Set Enrichment Analysis (GSEA) was performed. p values were assessed using 1000 permutations for each gene set and corrected with the false discovery rate (FDR) method. When analysing microarray data, the mean of all probes for the same gene was used to divide patients into high/low cohorts. The following publicly available data sets of PDAC patients were used for our analyses: NCBI-GEOGSE62452 and the data from Cao et al., 2021.

### Statistics

Graphs represent mean ± SD with each data point depicting a biological replicate, the n in the legends represents the number of biologically independent experiments. Statistical analysis was performed using the GraphPad Prism 9 software. For in vitro experiments, Gaussian distribution was assumed, and two-tailed parametric unpaired t-test was applied, while the non-parametric Mann-Whitney test was used for in vivo and ex vivo experiments. Data was considered as statistically significant if p < 0.05. For high throughput analysis (RNAseq, scioCyto Cytokine array) padj was calculated and results were considered significant if padj<0.05.

### Ovarian cancer: Database setup

The present inventors searched for ovarian cancer cohorts in NCBI Gene Expression Omnibus (https://www.ncbi.nlm.nih.gov/geo/) and in the Genomic Data Commons Data Portal (https://portal.gdc.cancer.gov/). Only samples with available transcriptome-level data with a minimum of ten patients were considered. To avoid differences due to different sensitivity, specificity, and dynamic range in detecting gene expression levels for specific genes by different technologies, the search was narrowed only to tumour samples examined using the *in situ* oligonucleotide array platforms GPL96 (Affymetrix Human Genome U133A Array), GPL571 (Affymetrix Human Genome U133A 2.0 Array), and GPL570 (Affymetrix Human Genome U133 Plus 2.0 Array). The advantage of these arrays is that they use identical probe sequences to measure the expression of individual genes.

### Ovarian cancer: Data processing

The oligonucleotide gene array files were MAS5 normalized. Then, a second scaling normalization was executed to set the mean expression in each array to 1000. Only the probes present in the GPL96 platform were used in the scaling normalization to prevent platform-specific differences due to the higher probe number in the GPL570 arrays. Quality control was executed by checking the background intensity, the noise, the percentage of present calls, the presence of bioBCD spikes, and the GAPDH/ACTB 3 to 5 ratio. JetSet package (https://services.healthtech.dtu.dk/services/jetset/) was used to select the most reliable probe set for each gene. Finally, cellular content for immune infiltration was determined using xCell (http://xCell.ucsf.edu/).

Clinical data were extracted from the supplemental material of the original publications or from the series matrix files in GEO. For each sample, overall survival (OS) time and event, progression-free survival (PFS) time and event, histological subtype, stage, grade, the success of debulking, TP53 mutation status, and treatment data were recorded whenever available. Debulking surgery, sometimes called ovarian cytoreduction surgery, is performed after the elimination of the ovaries and includes the removal of cancerous tumours from the pelvis. Generally, debulking surgery cannot lead to complete elimination of all tumour tissue, and the remaining cells can form a residual disease. For this reason, progression-free survival is published usually in ovarian cancer, which refers to time from treatment (surgery) to further disease progression in the patients.

### Ovarian cancer: Statistical analysis

Cox proportional hazards regression was used to compute differential survival. First, a univariate analysis was performed for each gene separately. To avoid missing correlations due to the use of a specific cutoff, all available cutoff values between the lower and upper quartiles of expression were used for each gene, and false discovery rate (FDR) using the Benjamini-Hochberg method was computed to correct for multiple hypothesis testing [19]. The cutoff value with the highest significance (lowest FDR) was determined - in case of multiple cutoff values with identical significance, the cutoff with the highest hazard (HR) rate was selected for the final analysis. When determining the top genes with the most reliable correlation to survival, only genes with a cutoff value over 200 were considered - this is twice the background intensity of about 100. In addition, preference was given to genes linked to worse prognosis with higher expression, as these genes represent optimal future therapeutic targets. For this selected set of top genes, multivariate Cox regression was computed to evaluate the effect on survival of clinical and pathological variables and gene expression. Kaplan-Meier plots were drawn to visualize survival differences using the cutoff values determined in the univariate analysis.

### Analysis platform extension

The previously established Kaplan-Meier plotter (https://www.kmplot.com) of the group of the present inventors was extended to include the complete integrated ovarian cancer database. The platform can be used to validate the findings of the present study in real time as well as for the future corroboration of new gene expression-based biomarker candidates and gene signatures in the present and additional sub-cohorts of patients not investigated in the current study.

### Example 1.- Verification of HAPLN1 role in peritoneal carcinomatosis in PDAC (Reference Example)

Given that different authors have reported different effects of HAPLN1 depending on the cancer type, the role of HAPLN1 in pancreatic ductal adenocarcinoma metastasizing was verified, considering hyaluronic acid (a component of the extracellular matrix) a crucial factor in regulating the acquisition of beneficial traits necessary for metastasis initiation such as cell plasticity, immune evasion, dormancy state control and organ colonization, which can be considered part of the processes of epithelial-to-mesenchymal transition (EMT) and stemness.

### 1.1. HAPLN1 accelerates peritoneal carcinomatosis in pancreatic ductal adenocarcinoma

The present inventors employed publicly available databases (NCBI-GEO GSE62452 and the data from Cao *et al.,* 2021*,*), where data corresponding to samples from PDAC tumours and also to the adjacent normal tissue that is usually resected together with the tumours (and which is frequently used as a healthy tissue referent with regard to the tumoral tissue) can be obtained. As explained in a previous publication (Wiedmann *et al.,* 2023), the inventors used a list of genes related to HA binding for the comparison between normal vs tumoral tissues. The inventors obtained that this list was enriched in tumour samples compared to normal tissue. Hyaluronan And Proteoglycan Link Protein 1 (HAPLN1) was the top contributor to the enrichment score, being the 8th most enriched gene overall. As explained above, patients were classified in HAPLN1 high or low, depending on whether their HAPLN1 expression levels were over or under the median of expression of the cohort. It was found that higher HAPLN1 expression correlated with shorter overall survival. When performing gene set enrichment analysis, in which a group of genes related with a certain function is use to compare in which of the two classes defined it is more expressed, the present inventors found several sets enriched in HAPLN1 high patients. Basal subtype signature, the group of genes defining the most malignant subtype of PDAC, and epithelial-to-mesenchymal signature, containing those genes that mediate the process, were both enriched in HAPLN1 high patients. Moreover, it was found that a list of genes expressed in peritoneal metastases was enriched in those patients with higher HAPLN1 expression, suggesting a higher risk for peritoneal carcinomatosis.

To study the role of HAPLN1 on PDAC in vitro, HAPLN1 was stably overexpressed in the murine PDAC cell line KPC. These cells are obtained from spontaneous tumours coming from the transgenic mice (KrasG12D; Trp53R172H; Elas-CreER). KPC-HAPLN1 cells were obtained by inducing the stable expression of the human HAPLN1 gene via lentiviral modification. KPC-HAPLN1 cells expressed more EMT markers, more stem-related genes and changed the proteoglycan production from Aggrecan to Versican, which is known to be pro-metastatic. It was also found that spheroid formation, a feature of stemness, was improved in KPC-HAPLN1 vs KPC. This was inferred by the capacity of cells to grow in the absence of a soil (low attachment) and to generate round and compact spheres. Additionally, embedding these spheroids into Matrigel, by the addition of cold Matrigel on top of the spheroid suspensions, led to an increased invasion of KPC-HAPLN1 cells. KPC-HAPLN1 cells improved KPC cell invasion capacities when co-cultured, indicating a paracrine effect.

For mouse experiments, female C57BU6J mice were ordered from Janvier (https://janvier-labs.com/en/) at 9-11 weeks of age. 1 million KPC or KPC-HAPLN1 cells expressing luciferase and RFP were injected intraperitoneally (i.p.) into mice. Intraperitoneal injection of luciferase expressing KPC cells resulted in higher luciferase activity when tumour cells expressed HAPLN1. Flow cytometry of the peritoneal lavage (PL) from these mice showed that there were significantly more tumour cells in KPC-HAPLN1 injected mice. Tumour cells and fibroblasts from the solid masses formed in these mice were isolated and RNA was extracted from them and analysed by RNAseq. RNAseq data of tumour cells isolated from tumour nodules and PL showed that KPC-HAPLN1 cells acquired an increased metastatic potential and a strong immunomodulatory phenotype. Thus, the immune cell composition of the PL was analysed by flow cytometry. Neutrophil and monocyte percentages were drastically reduced in KPC-HAPLN1 bearing mice. On the contrary, these mice had a significant increase in macrophages, which showed a reduction in pro-inflammatory gene expression.

All the above led to the conclusion that HAPLN1 expression in tumour cells promotes a hyperplastic phenotype that facilitates invasion and colonization of the peritoneum, among others by creation of a pro-tumoural immune microenvironment, thus confirming previous results.

### 1.2. HAPLN1 induced plasticity is mediated by TNFR2 signalling

TNF was identified as the most significant common upstream regulator and NFκB and TNF within the top 10 of genes with highest activation Z-score (Fig. 1A). In addition, when performing GSEA for the Hallmark for "TNFα signalling via NFκB", its enrichment in KPC-HAPLN1 tumour cells was confirmed (Fig. 1B).

These data suggest that HAPLN1 presence in tumours promoted TNFα signalling by the upregulation of the TNFR2 (the receptor whose gene is usually abbreviated as *Tnfrsflb*)*.*

In order to investigate whether TNFR2 regulation was a direct action of HAPLN1, the expression of the receptors TNFR1 (encoded by the gene *Tnfrsf1a*) and TNFR2 (encoded by the gene *Tnfrsf1b*) was analysed by qPCR in KPC-HAPLN1 vs. KPC. As shown in Fig. 2A, it was found that the differential regulation of the TNF receptors seen *in vivo* was reproduced *in vitro* in the absence of other cells, indicating that HAPLN1 directly induces *Tnfrsf1b* (TNFR2) expression. When analysing whether this receptor regulation could have an impact on TNFα responses (stimulation with 50 ng/ml recombinant TNFα for 6 h in starvation), it was found that HAPLN1 significantly increased the expression of *Tnfaip6* upon TNFα stimulation (Fig. 2B). Moreover, it was observed that stimulating cells with TNFα increased invasion (Fig. 2C), while inhibiting TNFα (by adding a TNFα antagonist, (10 µg/ml, ref. 654256-M, Merck Millipore, Germany, ) abolished HAPLN1-induced invasion (Fig. 2D).

In keeping with this, HAPLN1 significantly increased TNFα-dependent expression of Has2 (Fig. 2E). Interestingly, TNFα-induced invasion was dependent on HA synthesis (Fig. 2F), since cell incubation with TNFα together with the HAS inhibitor 4-methylumbelliferrone resulted in decreased invasion.

### Example 2.- Predictor capacity of gene signatures: TNFRSF1B, TNFAIP6, HAPLN1 and HAS2

### 2.1. Survival and gene expression: PDAC

As commented above, pancreatic cancer adenocarcinoma is the most lethal type of cancer. 5-year survival is very low (around 10%), mainly due to the metastatic potential of the tumours, what makes interesting to have available a way of identifying those patients that are in risk of developing metastasis. The vast majority of the transcriptomic data available, unfortunately, is at very early stages of tumour development, where tumours are still surgically removed.

The fact that HAPLN1 induced the expression of TNFRSF1B, which leads to the upregulation of HAS2 and TNFAIP6, prompted the present inventors to believe that the contribution of all of these genes would be similar to the contribution to each of them separately.

To explore the potential that these genes would have to diagnose those patients more prone to have metastasis after surgery, relapse free survival curves were prepared for said patients using the Kaplan-Meier plotter (https://kmplot.com/analysis/) with the data collected by this tool from public databases such as the one of TCGA. The high expression patients correspond to patients that have higher expression than the mean expression of all the patients, for all the genes of the signature. The KMplotter tool, analysing the clinical data provided by these databases, calculates the hazard ratio for the patients with higher expression of the signature to report a relapse after surgery. In the case of a multiple gene comparison, the tool will calculate a surrogate gene that represents the average expression level of all input genes.

Surprisingly, it was observed that the signature of the present invention (Fig. 3B) had a stronger correlation to survival compared to HAPLN1 alone (first graph of Fig. 3A, as indicated over the graph). Also surprisingly, the correlation was almost twice stronger than that obtained if the signature included only the genes TNFAIP6, HAPLN1 and HAS2 (Fig. 3C), a result that is particularly surprising because the Kaplan-Meier Plot corresponding to TNFRSF1B (last graph of Fig. A) shows a HR lower than 1, indicating a longer relapse free survival in those patients with higher expression, even though this is not significant (logrank P = 0.15, that is, higher than 0.01). This indicates that the signature of the present invention eliminates potential noise that each of these genes could generate, considering that it was made from bulk tissue. It is reasonable to speculate that the increase in expression of this gene, which is intimately related to immune infiltration, corresponds to a higher anti-tumour immunity in those patients. However, when considering the whole signature, this noise is reduced and the HR becomes higher than 1. In conclusion, the analysis of the signature is beneficial in terms of diagnosis because it can identify better those patients with a higher risk of relapse.

This is in accordance with the hazard ratio (HR) calculated for any of the individual genes, for only three of the genes (TNFAIP6, HAPLN1 and HAS2), and for the signature of the four genes (HAPLN1, HAS2, TNFAIP6 and TNFRSF1B). This is a very important value, because it indicates the risk of having a relapse instead of having a relapse-free survival (RFS): it is the factor by which the normal risk of having a relapse is multiplied, depending on the expression level, low or high. As can be seen in Fig. 3 and Table 2 below, for all of the genes, the hazard ratio of each gene was lower than that of the signature using the four genes together, which is higher than 4×10⁸, namely 4.6×10⁸. This is an unexpected and exceptional result, which indicates that not having, for the four genes, expression values higher the average expression obtained from the data of all the analysed patients is an excellent predictor of the risk of relapse, which risk will be low in that case, having a high probability of relapse free survival (RFS).

**Table 2**

| | Gene(s) | | | | | |
|---|---|---|---|---|---|---|
| | HAPLN1 | HAS2 | TNFAIP6 | TNFRSF 1B | TNFAIP6+ HAS2+ HAPLN1 | Signature (4 genes) |
| HR* | 3.34 (1.43 - 7.82) | 10.79 (1.45 - 80.57) | 3.85 (1.54-9.62) | 0.49 (0.18 - 1.33) | 7.08 (2.07 - 24.29) | 4.6×10⁸ (0-*∞*) |
| Logrank P | 0.0032 | 0.0038 | 0.002 | 0.15 | 0.0034 | 0.00067 |

| | | | | | | |
|---|---|---|---|---|---|---|
| *HR: Hazard Ratio | | | | | | |

The number of subjects at risk exhibiting high or low expression of each individual gene, all the genes of the group of TNFAIP6+HAS2+HAPLN1 (represented by the expression value of their calculated surrogate gene) or all the genes of the group of TNFAIP6+HAS2+HAPLN1+TNFRSF1B (the whole signature of the present invention, analogously represented by the expression value of their calculated surrogate gene) according to the time from the tumour resection (in months) can be found in Table 3 below:

**Table 3**

| | | Number of subjects at risk | | | | |
|---|---|---|---|---|---|---|
| | | Time (months) | | | | |
| Gene | Expression Level | 0 | 10 | 20 | 30 | 40 |
| HAPLN1 | Low | 51 | 39 | 18 | 14 | 8 |
| | High | 18 | 9 | 5 | 1 | 1 |
| HAS2 | Low | 18 | 13 | 9 | 7 | 3 |
| | High | 51 | 35 | 14 | 8 | 6 |
| TNFAIP6 | Low | 38 | 28 | 16 | 11 | 7 |
| | High | 31 | 20 | 7 | 4 | 2 |
| TNFRSF1B | Low | 45 | 28 | 14 | 10 | 8 |
| | High | 24 | 20 | 9 | 5 | 1 |
| TNFAIP6+ HAS2+HAPLN1 | Low | 29 | 21 | 14 | 10 | 6 |
| | High | 40 | 27 | 9 | 5 | 3 |
| Signature (four genes) | Low | 16 | 12 | 9 | 8 | 6 |
| | High | 53 | 36 | 14 | 7 | 3 |

Thus, it can be concluded that the signature including TNFRSF1B, TNFAIP6, HAPLN1 and HAS2 clearly defines those patients that were almost certainly going to develop metastasis.

### 2.2. Survival and gene expression: ovarian cancer

Subsequently, it was checked whether the identified signature could be valid for other abdominal tumours known to develop peritoneal carcinomatosis. In this sense, the same analysis explained above was carried out in patients suffering oesophageal, liver or cervical cancer, with very similar results. Considering the very different origin of each of these abdominal tumours, it appeared unlikely that they would share similar genetic traits. However, considering that these genes are related to the interaction with the extracellular matrix, it was decided to check it.

Ovarian cancer was chosen because, as explained previously, it is one of the cancers with the highest rate of patients developing peritoneal carcinomatosis. If left untreated every patient with ovarian carcinoma will develop peritoneal carcinomatosis.

In an attempt to validate this signature, HAPLN1 expression levels in ovarian cancer were analysed comparing them to PDAC. It was found that ovarian cancers had significantly more HAPLN1 expression (Fig. 4A). Since HAPLN1 have been found to be responsible for the expression of the other genes in the assays of the previous Example, it was not expectable that the signature would be better to classify patients compared to each gene alone.

For that, it was analysed whether these genes associated with a higher metastatic rate in patients. For that, using the KM-plotter website (*https:*//*kmplot.com*/*analysis*/), the present inventors selected patients that had an optimal debulking surgery, meaning that their tumour masses had been removed to a point at which no mass bigger than 1 cm² (detection limit) could be found. It was then analysed if the expression of all of these genes could predict those patients with higher risk to develop metastasis (progression free survival, PFS), finding that none of the genes alone was associated to a shorter PFS (Fig. 4B).

Surprisingly, when this analysis was repeated using the combined expression of all the genes, represented by a substitute (surrogate) value calculated by surrogate variable analysis, a very significant correlation with survival (progression free survival and overall survival) in ovarian cancer was found (Fig. 4C). Interestingly, even overall survival (OS) indicated that those patients with a surrogate value representing the expression of all of these genes higher than the mean expression of the cohort, (the surrogate gene which includes the expression of the four genes, calculated for the whole group of selected patients), had a worse prognosis.

Again, the hazard ratio (HR) value was very informative, being higher than 2 (2.19) when the signature of the four genes was analysed, as can be seen in Fig. 4 and Table 4 below. That indicates that the risk of relapse, particularly the risk of metastasis, was more than twice higher in those patients with a surrogate value representing the expression of all of these genes higher than the mean expression of the cohort. Moreover, the HR for HAPLN1 and HAS2 alone is below 1, what would indicate a longer relapse free survival for those patients with higher expression, reinforcing the benefit obtained with the signature of the present invention.

**Table 4**

| | Gene | | | | | |
|---|---|---|---|---|---|---|
| | HAPLN1 | HAS2 | TNFAIP6 | TNFRSF1B | Signature (PFS) | Signature (OS) |
| HR* | 0.86 (0.61 - 1.22) | 0.9 (0.73 - 1.12) | 1.42 (1.16 - 1.73) | 1.4 (1.13 - 1.73) | 2.19 (1.56 - 3.08) | 2.06 (1.27 - 3.34) |
| Logrank P | 0.41 | 0.34 | 0.00053 | 0.0015 | 3.1×10⁻⁶ | 0.0028 |

| | | | | | | |
|---|---|---|---|---|---|---|
| *HR: Hazard Ratio | | | | | | |

The values of number of subjects at risk in accordance with the gene expression, depending on the analysed gene (or combination of genes, that is, the signature of the present invention) can be found in Table 5 below:

**Table 5**

| | | Number of subjects at risk | | | | |
|---|---|---|---|---|---|---|
| | | Time from tumour resection (months) | | | | |
| Gene | Expression Level | 0 | 10 | 20 | 30 | 40 |
| TNFAIP6 | Low | 363 | 285 | 183 | 125 | 91 |
| | High | 333 | 226 | 135 | 81 | 51 |
| HAPLN1 | Low | 145 | 123 | 73 | 44 | 27 |
| | High | 95 | 74 | 50 | 35 | 28 |
| HAS2 | Low | 488 | 346 | 211 | 136 | 90 |
| | High | 208 | 165 | 107 | 70 | 52 |
| TNFRSF1B | Low | 514 | 377 | 247 | 159 | 112 |
| | High | 182 | 134 | 71 | 47 | 30 |
| Signature: PFS | Low | 135 | 117 | 84 | 51 | 37 |
| | High | 105 | 80 | 39 | 28 | 18 |
| Signature: OS | Low | 165 | 152 | 138 | 98 | 73 |
| | High | 78 | 73 | 60 | 45 | 33 |

Since these patients were all receiving chemotherapy as treatment, following the usual treatments for ovarian cancer (a platinum compound, usually cisplatin or carboplatin, and/or a taxane, such as paclitaxel or docetaxel), the present inventors decided to consider the possibility that perhaps those patients with higher expression of the signature of the present invention would respond worse to chemotherapy. The KM-plotter tool has another tool called ROC Plotter, which allows for the prediction of responders vs. non-responder patients to chemotherapy treatment. Interestingly, it was found that the signature of these four genes, represented by a surrogate value corresponding to their combined expression, also predict a bad response to chemotherapy, indicating that their expression is intimately correlated to plasticity also in ovarian cancer (Fig. 4D).

These data suggest that the gene signature proposed by the present invention could be a good predictor of those patients to whom traditional chemotherapy would not offer enough advantage. These patients could benefit from an alternative treatment that could improve their quality of life and their chances to beat the disease.

### References

Alsina-Sanchis, E. et al. Endothelial RBPJ Is Essential for the Education of Tumor-Associated Macrophages. Cancer Res. 82, 4414-4428 (2022).
Apte, M. V. et al. Extracellular matrix composition significantly influences pancreatic stellate cell gene expression pattern: role of transgelin in PSC function. AJP Gastrointest. Liver Physiol. 305, G408-G417 (2013).
Avula, L. R., Hagerty, B. & Alewine, C. Molecular mediators of peritoneal metastasis in pancreatic cancer. Cancer Metastasis Rev. (2020) doi:10.1007/s10555-020-09924-4.
Banerji, S. et al. Structures of the Cd44-hyaluronan complex provide insight into a fundamental carbohydrate-protein interaction. Nat. Struct. Mol. Biol. 14, 234-239 (2007).
Baranova, N. S. et al. Incorporation of pentraxin 3 into hyaluronan matrices is tightly regulated and promotes matrix cross-linking. J. Biol. Chem. 289, 30481-30498 (2014).
Baranova, N. S. et al. Inter-α-inhibitor impairs TSG-6-induced hyaluronan cross-linking. J. Biol. Chem. 288, 29642-29653 (2013).
Baranova, N. S. et al. The inflammation-associated protein TSG-6 cross-links hyaluronan via hyaluronan-induced TSG-6 oligomers. J. Biol. Chem. 286, 25675-25686 (2011).
Calvo, F. et al. Mechanotransduction and YAP-dependent matrix remodelling is required for the generation and maintenance of cancer-associated fibroblasts. Nat. Cell Biol. 15, 637-646 (2013).
Cabezas-Wallscheid, N. et al. Identification of regulatory networks in HSCs and their immediate progeny via integrated proteome, transcriptome, and DNA methylome analysis. Cell Stem Cell 15, 507-522 (2014).
Cao, X. Y. et al. Aberrant upregulation of KLK10 promotes metastasis via enhancement of EMT and FAK/SRC/ERK axis in PDAC. Biochem. Biophys. Res. Commun. (2018) doi: 1 0.1016/j.bbrc.2018.03.194.
Cao, L. et al. Proteogenomic characterization of pancreatic ductal adenocarcinoma. Cell 184, 5031-5052.e26 (2021).
Celià-Terrassa, T. & Kang, Y. Distinctive properties of metastasis-initiating cells. Genes Dev. (2016) doi:10.1101/gad.277681.116.
Coccolini, F. et al. Peritoneal carcinomatosis. World J. Gastroenterol. 19, 6979-6994 (2013).
Eble, J. A. & Niland, S. The extracellular matrix in tumour progression and metastasis. Clin. Exp. Metastasis (2019) doi:10.1007/s10585-019-09966-1.
Ecker, B. L. et al. Age-Related Changes in HAPLN1 Increase Lymphatic Permeability and Affect Routes of Melanoma Metastasis. Cancer Discov 9, 82-95 (2019).
Goossens, P. et al. Membrane Cholesterol Efflux Drives Tumour-Associated Macrophage Reprogramming and Tumour Progression. Cell Metab. 29, (2019).
Grover, J. & Roughley, P. J. The expression of functional link protein in a baculovirus system: analysis of mutants lacking the A, B and B' domains. Biochem. J. 300 ( Pt 2), 317-324 (1994).
Hanahan, D. Hallmarks of Cancer: New Dimensions. Cancer Discov. 12, 31-46 (2022). Joyce, J. A. & Pollard, J. W. Microenvironmental regulation of metastasis. Nat. Rev. Cancer 9, 239-252 (2009).
Massagué, J. & Obenauf, A. C. Metastatic colonization by circulating tumour cells. Nature 529, 298-306 (2016).
Matsumoto, K. et al. Distinct interaction of versican/PG-M with hyaluronan and link protein. J. Biol. Chem. 278, 41205-4121(2003).
Nie, X, et al. Prognostic signature of ovarian cancer based on 14 tumor microenvironment-related genes. Medicine (Baltimore) 2021 Jul 16;100(28):e26574. doi: 10.1097/MD.0000000000026574
Oohashi, T., Edamatsu, M., Bekku, Y. & Carulli, D. The hyaluronan and proteoglycan link proteins: Organizers of the brain extracellular matrix and key molecules for neuronal function and plasticity. Exp. Neurol. 274, 134-144 (2015)
Pearce, O. M. T. et al. Deconstruction of a metastatic tumor microenvironment reveals a common matrix response in human cancers. Cancer Discov. 8, 304-319 (2018).
Richter, R. P., Baranova, N. S., Day, A. J. & Kwok, J. C. Glycosaminoglycans in extracellular matrix organisation: are concepts from soft matter physics key to understanding the formation of perineuronal nets? Curr. Opin. Struct. Biol. 50, 65-74 (2018).
Seyfried, N. T. et al. Expression and purification of functionally active hyaluronan-binding domains from human cartilage link protein, aggrecan and versican: formation of ternary complexes with defined hyaluronan oligosaccharides. J. Biol. Chem. 280, 5435-5448 (2005).
Spicer, A. P., Joo, A. & Bowling, R. A. A Hyaluronan Binding Link Protein Gene Family Whose Members Are Physically Linked Adjacent to Chrondroitin Sulfate Proteoglycan Core Protein Genes: THE MISSING LINKS*. J. Biol. Chem. 278, 21083-21091 (2003).
Vennin, C. et al. Reshaping the Tumor Stroma for Treatment of Pancreatic Cancer. Gastroenterology (2018) doi:10.1053/j.gastro.2017.11.280.
Walker, C., Mojares, E. & Del Rio Hernández, A. Role of extracellular matrix in development and cancer progression. Int. J. Mol. Sci. (2018) doi:10.3390/ijms19103028.
Wiedmann, L. et al. HAPLN1 potentiates peritoneal metastasis in pancreatic cancer. Nat Commun 14, 2353 (2023). https://doi.org/10.1038/s41467-023-38064

## Claims

1. A method for the prognosis of the risk of developing metastasis, the survival time and/or the response to the administered chemotherapy in a subject under study suffering or having suffered from an abdominal cancer, comprising the steps of:
a) determining the expression value for each one of the genes of the group of TNFRSF1B, TNFAIP6, HAPLN1 and HAS2 in a tumour sample of the subject,
b) obtaining, by multivariate analysis, a first surrogate value which represents the expression value of a surrogate gene that represents the combined mean expression of aforementioned those four genes in the subject;
c) obtaining, by multivariate analysis, a second surrogate value that acts as reference value and which represents the mean expression value of a surrogate gene that represents the combined mean expression of the aforementioned four genes in a population of subjects suffering from the same cancer;
d) comparing the value obtained in b) with the value obtained in c),
e) concluding that the subject under study is:
i) at risk of developing metastases, and/or not responding to the administered chemotherapy, if the value obtained in b) for the subject is higher than the value obtained in c) for the population of cancer patients,
and/or
ii) at low risk of developing metastases or a high probability of not developing metastases, and/or is responding to the administered chemotherapy if the value obtained in b) for the subject is equal or lower than the value obtained in c) for the population of cancer patients.

2. The method according to claim 1, wherein the first value of b) and the second value of c) are determined by surrogate variable analysis.

3. The method according to any one of claims 1 or 2, wherein the subject is a human being.

4. The method according to any one of claims 1-3, wherein the level of expression of each of the genes TNFRSF1B, TNFAIP6, HAPLN1 and HAS2 is determined by determining the level of its messenger RNAs and/or the concentration of the protein expressed by the gene.

5. The method according to claim 4, wherein the level of expression of the genes is determined by determining the level of their messenger RNAs by qRT-PCR.

6. The method according to any one of the preceding claims, wherein the expression value for each one of the genes of the group of TNFRSF1B, TNFAIP6, HAPLN1 and HAS2 to be used in step b) and/or in step c) is determined in a tumour sample which is a biopsy.

7. The method according to any one of claims 1-5, wherein the expression values used to determine the second value of step c) are obtained from a public database related to cancer.

8. The method according to any one of the preceding claims, wherein the abdominal cancer is ovarian cancer or pancreatic cancer.

9. The method according to claim 8, wherein the abdominal cancer is pancreatic ductal adenocarcinoma (PDAC).

10. The method according to claim 9, wherein
a. the subject has suffered from PDAC, and
b. the first surrogate value which represents the expression value of a surrogate gene representing the combined mean expression of the four genes of the group of TNFRSF1B, TNFAIP6, HAPLN1 and HAS2 in the tumour sample of the subject, is equal or lower than the second surrogate value that acts as reference value for the expression of the four genes in the population of analysis,
and it is concluded that
i) the expected relapse free survival time for the subject is at least 48 months, and/or
ii) the probability of relapse free survival for the subject for at least 48 months is higher than that of a subject showing a surrogate value representing the combined mean expression of the four genes of the group of TNFRSF1B, TNFAIP6, HAPLN1 and HAS2 higher than the second surrogate value that acts as reference value for the expression of the four genes in the population of analysis.

11. The method according to claim 9, wherein
a. the subject has suffered from PDAC, and
b. the first surrogate value which represents the expression value of a surrogate gene representing the combined mean expression of the four genes of the group of TNFRSF1B, TNFAIP6, HAPLN1 and HAS2 in the tumour sample of the subject, is higher than the second surrogate value that acts as reference value for the expression of the four genes in the population of analysis, and it is concluded that
i) the expected relapse free survival time for the subject is equal or lower than 48 months.

12. The method according to claim 8, wherein
a. the subject has suffered from ovarian cancer, and
b. the first surrogate value which represents the expression value of a surrogate gene representing the combined mean expression of the four genes of the group of TNFRSF1B, TNFAIP6, HAPLN1 and HAS2 in the tumour sample of the subject is equal or lower than the second surrogate value that acts as reference value for the expression of the four genes in the population of analysis,
and it is concluded that
i) the subject has a probability of cancer progression-free survival more than 2 times higher than that of a subject showing a surrogate value representing the combined mean expression of the four genes of the group of TNFRSF1B, TNFAIP6, HAPLN1 and HAS2 higher than the second surrogate value that acts as reference value for the expression of the four genes in the population of analysis, and/or
ii) the subject has a probability of more than 40% of cancer progression-free survival during at least 48 months, and/or
iii) the subject has a probability of overall survival more than 2 times higher than that of a subject showing a surrogate value representing the combined mean expression of the four genes of the group of TNFRSF1B, TNFAIP6, HAPLN1 and HAS2 higher than the second surrogate value that acts as reference value for the expression of the four genes in the population of analysis, and/or
iv) the subject has a probability of more than 60% of survival during at least 48 months.

13. The method according to claim 8, wherein .
a. the subject has suffered from ovarian cancer, and
b. the first surrogate value which represents the expression value of a surrogate gene representing the combined mean expression of the four genes of the group of TNFRSF1B, TNFAIP6, HAPLN1 and HAS2 in the tumour sample of the subject, is higher than the second surrogate value that acts as reference value for the expression of the four genes in the population of analysis, and it is concluded that
i) the expected relapse free survival time for the subject is equal or lower than 48 months.
